(19) Europäisches Patentamt
European Patent Office
Office européen des brevets

(11) Publication number : 0 478 197 A1

(12) EUROPEAN PATENT APPLICATION

(21) Application number : 91308418.2

(22) Date of filing : 16.09.91

(51) Int. Cl.[5] : C07C 51/15, C07C 65/05, C07C 65/11

(30) Priority : 17.09.90 JP 244099/90

(43) Date of publication of application : 01.04.92 Bulletin 92/14

(84) Designated Contracting States : DE FR GB

(71) Applicant : MITSUI TOATSU CHEMICALS INC.
No. 2-5, Kasumigaseki 3-chome
Chiyoda-Ku Tokyo 100 (JP)

(72) Inventor : Nakanishi, Takehisa
3-7-5, Kamo
Takaishi-shi, Osaka-fu 592 (JP)
Inventor : Miura, Toshizumi
3-9-6-615, Nishitoriishi
Takaishi-shi, Osaka-fu 592 (JP)

(74) Representative : Hayward, Denis Edward Peter et al
Lloyd Wise, Tregear & Co. Norman House
105-109 Strand
London WC2R 0AE (GB)

(54) **Control process of isomer ratio of aromatic hydroxycarboxylic acid.**

(57) A process for carrying out a reaction of alkali metal phenolate with carbon dioxide in an organic phosphine oxide solvent and controlling the isomer ratio of resulting aromatic hydroxycarboxylic acids by changing the mole ratio of the organic phosphine oxide to the alkali metal phenolate in the range of from 1 to 4, is disclosed.

EP 0 478 197 A1

The present invention relates to a process for controlling isomer ratio of aromatic hydroxycarboxylic acids obtained in the preparation of aromatic hydroxycarboxylic acids by reacting an aromatic hydroxy compound with carbon dioxide. More particularly, the invention relates to the controlling process of isomer ratio in the preparation of aromatic hydroxycarboxylic acids by changing the ratio of organic phosphine oxide to alkali metal phenolates in a specific range.

Aromatic hydroxycarboxylic acids are useful compounds in industry as intermediates for fine chemicals and raw materials for resin.

Reaction of alkali metal phenolates with carbon dioxide has conventionally been known as Kolbe-Schmitt reaction which is carried out at high temperature under pressure in the absence of a solvent. In the reaction, carbon dioxide is absorbed in anhyhdrous sodium phenolate and heated to 120 ~ 140 °C to form mono- and di-sodium salicylate. When potassium phenolate is used and the reaction is carried out at high temperature, main product is p-hydroxybenzoic acid and salicylic acid is a byproduct (Organic chemical handbook, p 454, published from Gihodo Tokyo).

Hirao et al. have investigated the reaction in various solvents which include protic solvents such as tert-butanol; aprotic solvent such as toluene, diphenyl ether, dimethylformamide and dimethyl sulfoxide; and high-boiling solvents such as kerosene and light oil [ Yukigosei Kyokaishi, Vol. 24,p.1051(1966), Vol.25, p.412, 417, 577, 1031, 1202(1967), Vol.26, p.439, 992(1968), Vol.27, p.648(1969) and Vol.28, p.426(1970)].

Various hydroxynaphthalenecarboxylic acid can be obtained by reacting alkali metal naphtholate with carbon dioxide in a polar solvent [Journal of Chemical Society Japan 1989(7),1164].

Aromatic hydroxycarboxylic acids differ in the substitution positions of a hydroxyl group and carboxylic group. These compounds are useful in industry. Consequently, in the preparation of aromatic hydroxycarboxylic acids, for example, various kinds of hydroxybenzoic acid and hydroxynaphthalenecarboxylic acid, remarkable merit can be obtained in industrial production by optionally changing the proportion of isomers formed. However, a method for changing the resultant isomer ratio without increase in the by-product formation has not been known at all in the above synthetic methods.

An object of the invention is to provide a process for controlling the formation of aromatic hydroxycarboxylic acids so as to obtain the desired isomer ratio.

The present inventors have found that a carboxyl group is selectively introduced into the para position to the hydroxyl group of phenols by reacting an alkali metal phenolate with carbon dioxide in an organic phosphine oxide solvent(KOKAI TOKKYO KOHO Hei 2-22,354(1990)). As a result of further detailed investigation on the same reaction in the organic phosphine oxide solvent, the inventors have found that the isomer ratio of resulting aromatic hydroxycarboxylic acid can be controlled by changing the ratio of the organic phosphine oxide to the alkali metal phenolate in a specific range. Thus the invention has been completed.

That is, an aspect of the present invention is a preparation process of an aromatic hydroxycarboxylic acids by the reaction of an alkali metal phenolate with carbon dioxide in an organic phosphine oxide solvent comprising controlling the isomer ratio of resulting aromatic hydroxycarboxylic acids by changing the ratio of the organic phosphine oxide to the alkali metal phenolate.

According to the process of the invention, for example, in the case of reacting alkali metal phenolate with carbon dioxide, lower ratio of the organic phosphine oxide to the alkali metal phenolate leads to increase the amount of salicylic acid wherein a carboxyl group is introudced into the ortho position of the phenolic hydroxyl group. Higher ratio of the organic phosphine oxide causes increase in the formation of p-hydroxybenzoic acid wherein carboxyl group is introduced into the para position. When the ratio exceeds 4, the amount of p-hydroxybenzoic acid becomes almost constant. However, the selectivity improves only slightly, even though the ratio is further increased to 8. The ratio of 10 or more has little merit in production efficiency. On the other hand, when the ratio is less than 1, the alkali metal phenolate raw material insufficiently dissovles in the organic phosphine oxide and hence the reaction becomes difficult to progress.

The above phenomenon is generally observed in various kinds of the organic phosphine oxide. Consequently it is thought that the alkali metal phenolate and the organic phosphine oxide form a complex in the solution and the mode of complex formation determines the isomer ratio of the product.

The organic phosphine oxide which can be used in the invention is a compound represented by the formula (I):

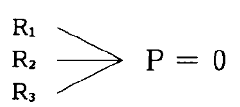

( I )

wherein $R_1$, $R_2$ and $R_3$ are individually a straight or branched alkyl group having from 1 to 8 carbon atoms, or

a phenyl group.

Exemplary organic phosphine oxides include
trimethylphosphine oxide, triethylphosphine oxide,
tri-n-propylphosphine oxide, triisopropylphosphine oxide,
tri-n-butylphosphine oxide, tri-sec-butylphosphine oxide,
tri-n-hexylphosphine oxide, tri-n-octylphosphine oxide,
dimethylethylphosphine oxide,
methyldiethylphosphine oxide,
diethylpropylphosphine oxide,
ethyldipropylphosphine oxide,
ethylpropylbutylphosphine oxide
and triphenylphosphine oxide.

Preferred phosphine oxide has same $R_1$, $R_2$ and $R_3$, and is a straight or branched trialkylphosphine oxide having from 1 to 8 carbon atoms or triphenylphosphine oxide.

More preferred phosphine oxide is selected from the group consisting of trimethylphosphine oxide, triethylphosphine oxide, tri-n-propylphosphine oxide, triisopropylphosphine oxide, tri-sec-butylphosphine oxide, tri-n-butylphosphine oxide, tri-n-hexylphosphine oxide and tri-n-octylphosphine oxide.

These organic phosphine oxides can be used singly or as a mixture. Triethylphosphine oxide (mp 50°C ) and tri-n-butylphosphine oxide (mp 67 ~ 69 °C ) are solid at room temperature. On the other hand an equal amount mixture of these oxides is liquid at room temperature, can be handled with ease, and maintains characteristics of each original phosphine oxide. Thus, the mixture can be used in place of single phosphine oxide.

Phenols which can be used for the alkali metal phenolate are phenol, cresols, monovalent phenols substituted with an alkyl group having from 2 to 12 carbon atoms, and monovalent phenols substituted with an aryl group having from 6 to 12 carbon atoms or naphthols. Exemplary phenols include, for example, phenol, cresol, ethylphenol, n-propylphenol, isopropylphenol, n-butylphenol, sec-butylphenol, tert-butylphenol, amylphenol, hexylphenol, octylphenol, nonylphenol, decylphenol, dodecylphenol, cyclohexylphenol, phenylphenol, ortho- or meta-substituted derivatives of these phenols, 1-naphthol and 2-naphthol.

By using these alkali metal phenolates as the raw material, aromatic hydroxycarboxylic acids can be obtained as main isomers. Phenols provide ortho substituted or para substituted aromatic hydroxycarboxylic acid wherein a carboxyl group is introduced into the ortho or para position to phenolic hydroxyl group.

1-Naphthol provides 1,2- or 1,4-substituted aromatic hydroxycarboxylic acid wherein a carboxyl group is introduced into the 2- or 4- position to the hydroxyl group at the 1-position.

2-Naphthol provides 2,3- or 2,6-substituted aromatic hydroxycarboxylic acid wherein a carboxyl group is introduced into the 3- or 6-position to the hydroxyl group at the 2-position. Practically, phenol gives p-hydroxybenzoic acid (hereinafter referred to as PHB) and salicylic acid (hereinafter referred to as SA) as main isomer products and the yields of these isomers can be controlled to the desired ratio. Formation of byproduct 4-hydroxyisophthalic acid (hereinafter referred to as OIP) can be inhibited.

Similarly, 1-hydroxynaphthalene gives 1-hydroxynaphthalene-2-carboxylic acid (hereinafter referred to as 1-HNC-2) and 1-hydroxynaphthalene-4-carboxylic acid (hereinafter referred to as 1-HNC-4) as main isomers and the yields of these isomers can be controlled to the desired ratio. Formation of another isomer 1-hydroxynaphtalene-5-carboxylic acid (hereinafter referred to as 1-HNC-5) can be inhibited. 2-Hydroxynaphthalene gives 2-hydroxynaphthalene-3-carboxylic acid (hereinafter referred to as 2-HNC-3) and 2-hydroxynaphthalene-6-carboxylic acid (hereinafter referred to as 2-HNC-6) as main isomers and the yields of these isomers can be controlled to the desired ratio. Formation of another isomer 2-hydroxynaphalene-1-carboxylic acid (hereinafter referred to as 2-HNC-1) can be inhibited.

Alkali metals which can be used for the preparation of the alkali metal phenolate include from light atoms such as lithium to heavy atoms such as sodium, potassium and rubidium. It is particularly favorable in economy that the isomer ratio of aromatic hydroxycarboxylic acids can be controlled in a broad range by using inexpensive raw materials such as sodium hydroxide.

The alkali metal phenolate used for the invention can be prepared by various methods. For example, an equivalent weight of an aqueous sodium hydroxide solution is added to phenol, evaporated to dryness, and substantially converted to the anhydrous alkali metal phenolate. More preferably, phenol is dissolved in an organic solvent, neutralized with an aqueous sodium hydroxide solution, and dried to give the alkali metal phenolate.

The alkali metal phenolate raw material can be readily dissolved in the above organic phosphine oxide in a hot state and is assumed to have a high efficiency in reaction with carbon dioxide and to accelerate the carboxylation reaction.

The ratio of the organic alkylphosphine oxide to the alkali metal phenolate is suitably determined depending

upon the prescribed isomer ratio of the aromatic hydroxycarboxylic acids. For example, the relationships between the ratio of triethylphosphine oxide to the alkali metal phenolate and the ratio of the yield of p-hydroxybenzoic acid to that of salicylic acid are previously examined. Then the required ratio of the oxide to the phenolate for obtaining the desired yield ratio is determined on the basis of the above relationships and the reaction is carried out by using the condition.

Preferred reaction temperature differs depending upon the kind of raw materials and reaction pressure, and is usually from 50 to 300 °C. The reaction temperature less than 50 °C decreases conversion of the alkali metal phenolate and unfavorably lowers reaction rate. Increased reaction temperature improves conversion of the alkali metal phenolate. However, the reaction temperature exceeding 300 °C is unfavorable because formation of dicarboxylic acid byproducts becomes remarkable.

No particular limitation is imposed upon the pressure of carbon dioxide. Preferred pressure is 1 atmosphere or more. Higher pressure leads to higher reaction rate and good results can be obtained. In order to achieve same degree of conversion, the reaction temperature can be decreased while increasing the reaction pressure. The reaction can, of course, be conducted above the critical temperature of carbon dioxide.

No particular restriction is placed on the reaction time. Extension of the reaction time can generally increase degree of conversion. Suitable reaction time varies depending upon the reaction temperature, kind of the solvent and other reaction conditions. The reaction usually completes within 12 hours.

Two methods are frequently used for the reaction. For example, in the frist method, the alkali metal phenolate is dissolved in a solvent, carbon dioxide is absorbed in the solution, and then the resultant mixture is heated to carry out carboxylation reaction.

In the second reaction, a mixture of the alkali metal phenolate and the solvent is heated to the presribed temperature and the reaction is carried out while blowing carbon dioxide through the mixture. Any method can be employed and the invention is not limited by these methods.

Compounds which are weakly coordinated with the alkali metal phenolate, for example, hydrocarbons have no influence on selectivity and may be present in the reaction, if desired. However, compounds such as water which strongly coordinate with or dissociate the phenolate are unfavorable for the reaction. Consequently, in carrying out the reaction, moisture must be restricted to 1 % or less, preferably 0.1 % or less in the reaction mixture.

The reaction of the invention can be carried out batchwise or continuously.

After finishing the reaction, water is added to the reaction mixture, aromatic hydroxycarboxylic acid salt is transfered into the aqueous layer by stirring, counter current extraction or other known procedures, and then separating the aqueous layer from the organic phosphine oxide layer. The separated aqueous layer is adjusted the pH by the addition of acid and each aromatic hydroxycarboxylic acid is precipitated isolatedly according to its acidity.

In order to increase partition from the reaction mixture to the aqueous layer in the above method, the second solvent can be added which is a good solvent for the organic phosphine oxide and simultaneously a bad solvent for water.

The invention will hereinafter be illustrated further in detail by way of examples. However, the scope of the invention is not limited by these examples.

In these examples, raw materials and products were analyzed by liquid chromatography (packing; silica gel $C_{1\ 8}$ eluate; acetonitrile-water)

Example 1

To a 200 $m\ell$ four necked glass flask equipped with a stirrer, carbon dioxide inlet tube and reflux condenser, 11.6g(100 m mol) of anhydrous sodium phenolate was charged and 53.7g (400 m mole) of triethyl phosphine oxide (hereinafter referred to as TEPO) which was previously dried with molecular sieve was added and heated to 50 °C to obtain a solution. Then carbon dioxide was blown through the solution and sufficiently absorbed at 50 °C with stirring under atmospheric pressure. Thereafter the internal temperature of the reaction vessel was increased to 140 °C over 10 minutes while continuing the blowing of carbon dioxide. Carbon dioxide was further blown through the reaction mixture at 140 °C for an hour under atmosheric pressure to continue the reaction.

According to the analysis of reaction mass by liquid chromatography, conversion of sodium phenolate was 30 %, and selectivity of PHB and SA was 90 % and 9 %, respectively. Trace OIP was also detected.

Results are illustrated in Table 1.

To the reaction mass, 60 g of water and 30 g of toluene were added, shaken, allowed to stand, and an aqueous layer was separated from an organic layer. Thus the resulting organic acid salt was transfered to the aqueous layer. p-Hydroxybenzoic acid was separated by adjusting the aqueous layer to pH 4.0 with addition of sulfuric acid. p-Hydroxybenzoic acid is filtered and the resulting filtrate was adjusted to pH 1.5 by further

adding sulfuric acid. Thus salicylic acid was precipitated. p-Hydroxybenzoic acid and salicylic acid thus obtained had purity of 99 %, respectively. These products had sufficiently high purity as technical grade.

Example 2

The same procedures as described in Example 1 were carried out except that the mole ratio of TEPO to sodium phenolate was 2.

Results are illustrated in Table 1

Example 3

The same procedures as described in Example 1 were carried out except that the mole ratio of TEPO to sodium phenolate was 1.
Results are illustrated in Table 1.

Examples 4 and 5

The same procedures as described in Example 1 were carried out except that tributylphosphine oxide was used in place of TEPO and the mole ratio of tributyl phosphine oxide to sodium phenolate was 4 and 1 in Examples 4 and 5, respectively.

Results are illustrated in Table 1

Examples 6 ~ 9

The same procedures as described in Example 1 were carried out except that a SUS 316 stainless autoclave was used as a reaction vessel, triphenylphosphine oxide or tributylphosphine oxide was used in place of TEPO, and the reaction was carried out under pressure.

Results are illustrated in Table 1.

Examples 10 ~ 12

The same procedures as described in Examples 1, 2 and 3 were carried out, respectively, except that potassium phenolate was used in place of sodium phenolate. Results are illustrated in Table 2.

Examples 13 and 14

A SUS autoclave was used as reaction vessel. Triethylphosphine oxide was used as a solvent. Sodium phenolate, was used as a raw material. The reaction was carried out at 60 °C for an hour under carbon dioxide pressure of 90 $kg/cm^2G$. The mole ratio of triethylphosphine oxide (TEPO) to sodium phenolate was 4 and 1 in Examples 13 and 14 respectively. Results are illustrated in Table 2.

Comparative Example 1

The same procedures as described in Example 13 was carried out except that the mole ratio of triethylphosphine oxide to sodium phenolate was 8.

Results are illustrated in Table 2.

The ratio of resulting p-hydroxybenzoic acid to salicylic acid is about the same as in the case of 4 mole ratio. Even though the mole ratio of triethylphosphine oxide to sodium phenolate was varied, the ratio of the resulting isomers was almost the same.

Examples 15 and 16

Tributylphosphine oxide was used as the solvent. Sodium o-cresolate was used as a raw material. The reaction was carried out at 140 °C for an hour under carbon dioxide pressure of 10 $kg/cm^2G$. The mole ratio of tributylphosphine oxide to sodium o-cresolate was 4 and 1, in Examples 15 and 16, respectively. Results are illustrated in Table 3.

Examples 17 and 18

Anhydrous sodium m-cresolate was added to an equimolar solvent mixture of tributylphosphine oxide and trioctylphosphine oxide. Then carbon dioxide was blown through the solution. The reaction was carried out at 120°C for 3 hours under atmospheric pressure of carbon dioxide. The mole ratio of trioctylphosphine oxide to sodium m-cresolate was 4 and 2 in Examples 17 and 18, respectively. Results are illustrated in Table 3.

Comparative Example 2

The same procedures as described in Example 17 were carried out except that the mole ratio of the equimolar solvent mixture to sodium m-cresolate was 8.

Results are illustrated in Table 3.

The ratio of resultant PHB and SA was about the same as in the case of 4 mole ratio. Even though the mole ratio of the solvent mixture to sodium m-cresolate was varied, the ratio of resulting isomers was almost the same.

Examples 19 and 20

Anhydrous potassium o-phenylphenolate was dissolved in tributylphosphine oxide and reacted at 100 °C for an hour under atmospheric pressure of carbon dioxide.

Results are illustrated in Table 3.

Examples 21 and 22

Tributylphosphine oxide was used as a solvent. Anhydrous sodium 1-naphtholate was used as a raw material. The reaction was carried out at 200 °C for 2 hours under the carbon dioxide pressure of 10 kg/cm$^2$G. The mole ratio of tributylphosphine oxide to sodium 1-naphtholate was 4 and 2 in Examples 21 and 22, respectivley.

Results are illustrated in Table 4.

The reaction mass was analyzed by liquid chromatography. Main products were 1-HNC-2 and 1-HNC-4. A small amount of 1-HNC-5 was detected. The equal amount of water to tributylphosphine oxide was added to the reaction mixture and formed carboxylic acid salts were extracted to the aqueous layer. Sulfuric acid was added by small portions to the aqueous layer to precipitate 1-HNC-4. 1-HNC-4 was separated by filtration. Sulfuric acid was further added to the resulting filtrate to precipitate and separate 1-HNC-2.

Examples 23 and 24

Triethylphosphine oxide was used as a solvent. Anhydrous potassium 2-naphtholate was used as a raw material. The reaction was carried out at 260°C for an hour under the carbon dioxide pressure of 30 kg/cm$^2$G. The mole ratio of triethylphosphine oxide to potassium 2-naphtholate was 4 and 2 in Examples 23 and 24, respectively. Results are illustrated in Table 5. The reaction mass was analyzed by lilquid chromatography. Main products were 2-HNC-3 and 2-HNC-6.

A small amount of 2-HNC-1 was detected.

Comparative Example 3

The same procedures as described in Example 23 were carried out except that the mole ratio of triethylphosphine oxide to potassium 2-naphtholate was 5.

The results are illustrated in Table 5.

Table 1

| Example | TRPO*1 | ArOM*2 | TRPO*1 / ArOM*2 (mole ratio) | Reaction condition Temp. (℃) | Pressure ($kg/cm^2$-G) | Time (hr) | Conversion (%) | Selectivity (%) P H B | S A |
|---|---|---|---|---|---|---|---|---|---|
| xample 1 | TEPO*3 | PhONa*6 | 4 | 140 | atmos-pheric | 1 | 30 | 90 | 9 |
| xample 2 | ↑ | ↑ | 2 | ↑ | ↑ | ↑ | 22 | 60 | 39 |
| xample 3 | ↑ | ↑ | 1 | ↑ | ↑ | ↑ | 15 | 44 | 56 |
| xample 4 | TBPO*4 | ↑ | 4 | ↑ | ↑ | ↑ | 22 | 80 | 18 |
| xample 5 | ↑ | ↑ | 1 | ↑ | ↑ | ↑ | 8 | 41 | 58 |
| xample 6 | TPPO*5 | ↑ | 4 | 150 | 10 | 3 | 38 | 53 | 46 |
| ample 7 | ↑ | ↑ | 2 | ↑ | ↑ | ↑ | 23 | 34 | 65 |
| ample 8 | TBPO*4 | ↑ | 4 | 100 | ↑ | 6 | 42 | 90 | 9.5 |
| ample 9 | ↑ | ↑ | 2 | ↑ | ↑ | ↑ | 11 | 65 | 35 |

Note:
*1 Organic phosphine oxide
*2 Alkali metal phenolate
*3 Triethylphosphine oxide
*4 Tributylphosphine oxide
*5 Triphenylphosphine oxide
*6 Sodium phenolate

Table 2

| Example | TRPO*1 | ArOM*2 | TRPO*1 / ArOM*2 (mole ratio) | Reaction condition | | | Conversion | Selectivity (%) | |
|---|---|---|---|---|---|---|---|---|---|
| | | | | Temp. (℃) | Pressure (kg/cm² -G) | Time (hr) | (%) | PHB | SA |
| Example 10 | TEPO*1 | PhOK*7 | 4 | 140 | atmospheric | 1 | 32 | 96 | 4 |
| Example 11 | ↑ | ↑ | 2 | ↑ | ↑ | ↑ | 23 | 62 | 37 |
| Example 12 | ↑ | ↑ | 1 | ↑ | ↑ | ↑ | 16 | 45 | 55 |
| Example 13 | ↑ | PhONa*6 | 4 | 60 | 90 | ↑ | 39 | 97 | 2 |
| Example 14 | ↑ | ↑ | 1 | ↑ | ↑ | ↑ | 24 | 48 | 51 |
| Comparative Example 1 | ↑ | ↑ | 8 | ↑ | ↑ | ↑ | 42 | 97 | 2 |

Note: *1 Organic phosphine oxide
*2 Alkali metal phenolate
*3 Triethylphosphine oxide
*6 Sodium phenolate
*7 Potassium phenolate

Table 3

| Example | TRPO*1 | ArOM *2 | TRPO*1 / ArOM*2 (mole ratio) | Reaction condition: Temp. (℃) | Reaction condition: Pressure ($kg/cm^2$-G) | Reaction condition: Time (hr) | Conversion (%) | Selectivity (%): PHB | Selectivity (%): SA |
|---|---|---|---|---|---|---|---|---|---|
| Example 15 | TBPO *4 | o-CrONa *9 | 4 | 140 | 10 | 1 | 50 | 93 | 6 |
| Example 16 | ↑ | ↑ | 1 | ↑ | ↑ | ↑ | 41 | 54 | 45 |
| Example 17 | TBPO *4 / TOPO*8 | m-CrONa *10 | 4 | 120 | atmospheric | 3 | 37 | 78 | 22 |
| Example 18 | ↑ | ↑ | 2 | ↑ | ↑ | ↑ | 26 | 49 | 51 |
| Comparative Example 2 | ↑ | ↑ | 8 | ↑ | ↑ | ↑ | 38 | 79 | 21 |
| Example 19 | TBPO*4 | O-PhPhOK *11 | 4 | 100 | ↑ | 1 | 27 | 90 | 9 |
| Example 20 | ↑ | ↑ | 2 | ↑ | ↑ | ↑ | 18 | 68 | 31 |

Note:
*1 Organic phosphine oxide
*2 Alkali metal phenolate
*4 Tributylphosphine oxide
*8 Trioctylphosphine oxide
*9 Sodium o-cresolate
*10 Sodium m-cresolate
*11 Potassium o-phenylphenolate

Table 4

| Example | TRPO*1 | ArOM*2 | TRPO*1 / ArOM*2 (mole ratio) | Reaction condition | | | Conversion (%) | Selectivity (%) | |
|---|---|---|---|---|---|---|---|---|---|
| | | | | Temp. (℃) | Pressure ($kg/cm^2$-G) | Time (hr) | | 1-HNC-2 | 1-HNC-4 |
| Example 21 | TBPO *4 | 1-NONa *12 | 4 | 200 | 10 | 2 | 47 | 32 | 58 |
| Example 22 | ↑ | ↑ | 2 | ↑ | ↑ | ↑ | 30 | 66 | 23 |

Note: *1 Organic phosphine oxide
*2 Alkali metal phenolate
*4 Tributylphosphine oxide
*12 Sodium 1-naphtholate

Tabie 5

| Example | TRPO*1 | ArOM*2 | TRPO*1 / ArOM*2 (mole ratio) | Reaction condition Temp. (℃) | Reaction condition Pressure (kg/cm² -G) | Reaction condition Time (hr) | Conversion (%) | Selectivity (%) 2-HNC-3 | Selectivity (%) 2-HNC-6 |
|---|---|---|---|---|---|---|---|---|---|
| Example 23 | TEPO *3 | 2-NONa *13 | 4 | 260 | 30 | 1 | 33 | 33 | 63 |
| Example 24 | ↑ | ↑ | 2 | ↑ | ↑ | ↑ | 24 | 59 | 38 |
| Comparative Example 3 | ↑ | ↑ | 5 | ↑ | ↑ | ↑ | 35 | 34 | 63 |

Note: *1 Organic phosphine oxide
*2 Alkali metal phenolate
*3 Triethylphosphine oxide
*13 Potassium 2-naphtholate

## Claims

**1)** A process for controlling the isomer ratio of aromatic hydroxycarboxylic acids in the reaction of an alkali metal phenolate with carbon dioxide in an organic phosphine oxide solvent comprising changing said isomer ratio by varying the mole ratio of the organic phosphine oxide to the alkali metal phenolate in the range of from 1 to 4.

**2)** The process of claim 1 wherein the organic phosphine oxide is a single compound or a mixture of the compound selected from an organic phosphine oxide represented by the formula (I ):

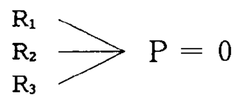

( I )

wherein $R_1$, $R_2$ and $R_3$ are individually a straight or branched alkyl group having from 1 to 8 carbon atoms, or a phenyl group.

**3)** The process of claim 1 or claim 2 wherein the phenol is a monoaromatic phenol and the aromatic hydroxycarboxylic acids are 2-hydroxyphenyl-1-carboxylic acid and 4-hydroxyphenyl-1-carboxylic acid.

**4)** The process of claim 3 wherein the monoaromatic phenol is a phenol compound selected from the group consisting of phenol, cresol, monovalent phenols substituted with an alkyl group having from 2 to 12 carbon atoms, and monovalent phenols substituted with an aryl group having from 2 to 12 carbon atoms.

**5)** The process of claim 3 wherein the monoaromatic phenol is phenol.

**6)** The process of claim 1 or claim 2 wherein the phenol is 1-naphthol and the aromatic hydroxycarboxylic acids are 1-hydroxynaphthane-2-carboxylic acid and 1-hydroxynaphthalene-4-carboxylic acid.

**7)** The process of claim 1 or claim 2 wherein the phenol is 2-naphthol and the aromatic hydroxycarboxylic acids are 2-hydroxynaphthalene-3-carboxylic acid and 2-hydroxynaphthalene-6-carboxylic acid.

European Patent Office

# EUROPEAN SEARCH REPORT

Application Number EP 91308418.2

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A<br><br><br>D | EP - A - 0 371 721<br>(MITSUI TOATSU)<br>* Claims; examples *<br>& JP-A-2-223 540<br>-- | 1-5 | C 07 C 51/15<br>C 07 C 65/05<br>C 07 C 65/11 |
| A | DE - A - 2 837 053<br>(K.K. UENO SEIYAKU OYO KENKYUJO)<br>* Claims *<br>---- | 1,6,7 | |
| | | | TECHNICAL FIELDS SEARCHED (Int. Cl.5) |
| | | | C 07 C 51/00<br>C 07 C 65/00 |

The present search report has been drawn up for all claims

| Place of search | Date of completion of the search | Examiner |
|---|---|---|
| VIENNA | 17-12-1991 | HOFBAUER |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)